# EUROPEAN PATENT SPECIFICATION

(11) **EP 2 087 897 B1**
(45) Date of publication and mention of the grant of the patent: **16.11.2011**
(21) Application number: 07866932.2
(22) Date of filing: 09.10.2007
(51) Int. Cl.: A61K 35/60, A61P 37/04, A61K 31/711

(54) **METHOD FOR PRODUCING APYROGENIC IMMUNOMODULATOR**
VERFAHREN ZUR HERSTELLUNG EINES APYROGENEN IMMUNMODULATORS
PROCÉDÉ DE PRODUCTION D'UN IMMUNOMODULATEUR APYROGÉNIQUE

(30) Priority: 03.11.2006 RU 2006138863
(43) Date of publication of application: 12.08.2009
(73) Proprietor: Kaplina, Elli Nikolaevna, Moscow 101000 (RU); Ladyigin, Alexandr Evgenevich, Moscow 101000 (RU); Kaplin, Valeri Yurievich, Moscow 119136 (RU)
(72) Inventor: Kaplina, Elli Nikolaevna, Moscow 101000 (RU); Ladyigin, Alexandr Evgenevich, Moscow 101000 (RU); Kaplin, Valeri Yurievich, Moscow 119136 (RU)
(74) Representative: Benatov, Emil Gabriel
(86) International application number: PCT/RU2007/000547
(87) International publication number: WO 2008/054247

(56) References cited:
- RU-A- 93 018 605
- RU-C1- 2 005 724
- RU-C1- 2 017 496
- RU-C1- 2 172 632
- RU-C1- 2 236 853
- NOSSIK D ET AL: "A Fe<3+>/DNA complex induces an anti-human immunodeficiency virus factor(s) in CD4+ lymphocyte cell lines" ACTA VIROLOGICA 199902 SK, vol. 43, no. 1, February 1999 (1999-02), pages 25-30, XP009110779 ISSN: 0001-723X

## Description

### Field of invention

The invention relates to the novel high-performance method for producing apyrogenic immunomodulator, based on low-molecular sodium deoxyribonucleate.

The invention can be used in chemical-pharmaceutical industry and biotechnology and the produced medicinal preparation, an immunomodulator, can be used in medicine, animal medicine, perfumery, cosmetology, research practice, etc.

The task of present invention is to develop the industrial high-performance method for producing a pharmaceutical preparation based on sodium salt of deoxyribonucleic acid, meeting the strict current requirements of therapeutic efficiency and safety for pharmaceutical preparations, and also having high technological parameters, in particular minimal losses, optimal reagent concentration, giving the possibility to decrease the adverse environmental impact of production process. In formulating this task the current requirements for organization of corresponding production processes, concerning the prevention of the any microbial contamination of the product during the process, and the circumstance that the medicinal preparation production should be carried out under aseptic conditions were taken into account. The preparations based on sodium deoxyribonucleate in aqueous solution of salt are produced in aseptic form with using conserving agent.

The content of ballast substances and impurities in pharmaceutical preparations is strictly limited by the international rules of pharmaceutical preparation production.

### Background of the invention

A number of methods for producing medicinal preparations based on sodium deoxyribonucleate are known. The main disadvantage of most of them is that these methods cannot meet the strict current requirements for pharmaceutical productions and medical equipment and also strict requirements of efficiency and safety for medicinal preparations.

The method for producing DNA sodium salt (Patent RU 61 K 35/60, C 07 H 21/04 No. 2017496, 1990) is known, the essence of which is to increase the yield of the desired product. However, the produced product comprises the high content of protein (1.5%), polysaccharides (2%) and RNA (2%). These impurities cause pyrogenic reactions, which are inconsistent with the basic requirement of safety application for medicinal preparations. An injectional form of preparation based on isolated DNA sodium salt can't be produced according with this patent because of high pyrogenicity.

The method for producing a sterile solution of native sodium salt of deoxyribonucleic acid in sodium chloride solution (Patent RU C 07 H 21/04 No. 2055837, 1996) is known. This method uses double ultrasonic treating, however the produced product is characterized by increased content of impurities. The end product contains the high amount of proteins (0.09 g/L) and other ballast substances and therefore cannot be used for medicinal preparation production. The sterile solution according with this patent is unsuitable for intravenous administration of preparation.

The method for producing the sodium salt of deoxyribonucleic acid from raw animal material and a apparatus for its embodiment (Patent RU C 07 P 21/04 No. 2005724, 1993) are known. However, the technology of product production according with this patent does not meet the all current requirements for biotechnological production and the produced product cannot be used to produce preparations for intravenous administration because of the high content of protein and polysaccharides causing pyrogenic reactions in the body.

A method for producing an immunotropic sterile apyrogenic tolerant preparation based on a sodium salt of the native low-molecular deoxyribonucleic acid (Patent RU C 07 H 21/04 No. 2236853, 2003) is closest to the claimed invention. However, the produced preparation cannot be used for intravenous administration because of a number of parameters (nativity index, allowable impurities, pH, etc.).

### Essence of invention

We have surprisingly found that assigned task can be solved and desired technical result can be reached by using the technological parameters stated below.
1. Carrying out the multiple stage of homogenization at high rate of homogenizer (12,000 rpm) with centrifuging in a refrigerated sedimentation centrifuge at 3,500 rpm causes destruction of milt roe cells and isolation of the nuclear mass, nearly complete separation of ribonucleic acid (RNA) and fatty acids. The said rates allow the necessary material to be obtained in maximum quantity without its destruction.
2. Additional incorporation of the calculated quantity of sodium dodecyl sulfate (SDS) after three or four cycles of homogenization, centrifugation and decantation of the homogenate, centrifugation being carried out at increased rate of centrifuge rotation (3,500 rpm) to produce an air-fat foam, redounding to complete isolation of residual fat mass, polysaccharides and RNA.
3. Incorporation of solution of sodium dodecyl sulfate in ethanol at the best ratio causes total lysis of nuclear envelopes to form the DNA-Na-detergent-protein complex.
4. Exposing the purified reaction mass to ultrasound at a vibration frequency of 21.7 to 23 kHz, vibration amplitude of at least 9 µm, generator capacity of 1.5-2 kW for a given time, ensures the high yield of DNA-Na with given range of molecular weight fragments and the nativity index of more than two.
5. The narrow range of pH (7.9-8.0) during filtration redounds to the complete isolation of protein compounds due to the preservation of native protein structure.
6. Drying the substance at 40-60°C in a calculated laminar flow of sterile air excludes the appearance of bacterial endotoxins and increases the purity class of the obtained product.

The claimed method is carried out as follows. The raw animal material (salmon or sturgeon milt roe) is defrosted, purified, degreased and homogenized in a citric-salt solution with nuclei lysis as the result. The homogenate is centrifuged in a refrigerated sedimentation centrifuge, and the supernatant is decanted and removed. The obtained substrate is then homogenized many times, centrifuged (to produce air-fat foam) and decanted (in the interim, lysis of nuclear envelopes and formation of the DNA-Na-detergent-protein complex happen). The purified homogenate is treated with ethanol solution of detergent for 2 hours and then with a strong solution of sodium chloride for 1.5 hours at elevated temperature; (the complex is broken down, DNA remains in the solution and the protein precipitates). The reaction mixture is then cooled to 10-15°C and exposed to ultrasound for more than 80 min in baths with piezoceramic radiators operating at vibration frequency of 21.7 to 23 kHz, vibration amplitude of up to 12 µm and generator capacity of 1.5-2 kW to ensure the molecular weight fragmentation of DNA-Na. The sonicated mass is mixed with kieselgur at the ratio of 10:2 to 30:2 (by weight) (to separate DNA-Na from residual protein). The liquid phase is separated by filtration, and the filter cake is additionally washed with permeate (obtained at the stage of concentration). The total filter liquor is concentrated by baromembrane filtration, and the concentrate is filtered through a sterilizing filter. Sodium deoxyribonucleate is precipitated from the concentrate by ethanol, and the precipitate is dried under aseptic conditions at 40-60°C for 4 hours in continuous flow of calculated quantity of sterile air mixed with nitrogen, supplied in drying chamber. To obtain an apyrogenic immunomodulator, the calculated amount of the obtained substance is dissolved in isotonic solution of sodium chloride.

The aseptic conditions - leak-tight compartment with controlled parameters of air: air change per hour of at least 20, temperature of 21-24°C, dust content of no more then 350,000 particles/m3 (C zone) and of no more then 3,500 particles/m3 (A zone), microorganism content of no more then 100 CFU/m3 (C zone) and of no more then 1 CFU/m3 (A zone).

According with present invention the yield of the sterile pharmaceutical substance of sodium deoxyribonucleate in form of amorphous white odorless powder, which is very soluble in aqueous solutions of sodium chloride, is up to 7.5% of the loaded milt roe. This pharmaceutical substance has the basic substance matter content of at least 99.6 to 100% and the ballast substances in the following amounts:
protein: up to 0.1 %
RNA: up to 0.2%
polysaccharides: up to 0.1%

The residual moisture content of pharmaceutical substance is 5-5.5% and the hyperchromic effect of pharmaceutical substance is over 42%.

The substance is used to produce the sterile solutions containing 15 mg/mL of sodium deoxyribonucleate in 0.9-0.95% aqueous solution of sodium chloride.

Controlled molecular weight, preservation of secondary structure and high degree of purification ensure high biological and immunotropic activities and prolonged stability of the medicinal preparation.

The invention is illustrated by means of the specific embodiments, shown below:
**Example 1.** 1 kg of purified salmon milt roe is disintegrated in a meat chopper, homogenized in 2 L of a citric-salt solution at 12,000 rpm. Then the homogenate is centrifuged in a refrigerated sedimentation centrifuge at 3,500 rpm, and the supernatant is decanted and removed. The obtained substrate is again homogenized in 2 L of the citric-salt solution with subsequent centrifugation and decantation of the supernatant three times, the homogenate is charged into a vessel with a warm citric-salt solution (7 L) and 3% solution of sodium dodecyl sulfate in 45% ethanol (1.9 kg) is added. The reaction mass is incubated at 60°C for 1.5 hours under continuous agitation. 5M sodium chloride solution (14.1 kg) is then added and the mixture is agitated at the same temperature for further 1.5 hours. Following this, the reaction mass is cooled to 10-15°C. The cooled mass (26 kg) is exposed to ultrasound in baths with piezoceramic radiators operating at vibration frequency of 21.7 to 23 kHz, vibration amplitude of 9 µm and generator capacity of 1.5-2 kW for 85 min. The sonicated mass is then mixed with kieselgur at a ratio of 10:2 (by weight). To separate DNA-Na from protein, the liquid phase is then separated using a suction filter, the filter cake is washed with permeate (10 L) and combined with the filtrate. Following additional microfiltration, the filtrate is concentrated (by the baromembrane method) on a membrane with a pore size of 0.04 µm. This yields 5 kg of the concentrated product containing 2.0% (by weight) of sodium deoxyribonucleate. The desired product is precipitated from the concentrate by ethanol. The precipitate is separated by centrifugation and dried at 40°C for 4 hours.

This yields 70 g of sodium deoxyribonucleate having the following characteristics: molecular weight of 480 kDa, protein content of 0.1%, RNA + polysaccharides content of 0.2%, moisture of 5%, hyperchromic effect of 44%. The calculated amount of the powder, needed to prepare a preparation concentration of 1.5%, is weighed out under aseptic conditions and under continuous flow of sterile dried air supplied into a drying chamber and dissolved in a 0.9% sterile solution of sodium chloride. The resulting solution is then aseptically poured into vials in therapeutic doses.

The analytical passport of the obtained preparation:
Identity - conforms;
Chromacity - colorless;
Transparency - conforms;
Sterility - sterile;
Toxicity - nontoxic;
Pyrogenicity - apyrogenic;
Nativity index - 2.4;
Content of sodium deoxyribonucleate - 0.016 g/mL;
pH - 8;
Content of sodium chloride - 0.9%;
Hyperchromic effect - 44%;
RNA content - 0.2%;
Protein content - 0.1 %.

**Example 2.** 1 kg of purified sturgeon milt roe is disintegrated in a meat chopper, homogenized in 2 L of a citric-salt solution at 12,000 rpm. The homogenate is then centrifuged in a refrigerated sedimentation centrifuge at 3,500 rpm, and the supernatant is decanted and removed. The substrate is thrice homogenized in 2 L of the citric-salt solution with subsequent centrifugation and decantation of the supernatant. Following this, the homogenate is charged into a vessel with a warm citric-salt solution (7 L) and 6% solution of sodium dodecyl sulfate in 45% ethanol (1.9 kg) is added. The reaction mass is incubated at 60°C for 1.5 hours under continuous agitation. Following this, 5M sodium chloride solution (14.1 kg) is added and the mixture is agitated at the same temperature for further 1.5 hours. Following this, the reaction mass is cooled to the temperature, which is not more 15°C, and exposed to ultrasound in baths with piezoceramic radiators operating at a vibration frequency of 21.7 to 23 kHz, vibration amplitude of 11 µm and generator capacity of 1.5-2 kW for 90 min. The sonicated mass is then mixed with kieselgur at a ratio of 30:2 (by weight). The liquid phase is separated using a suction filter. The filter cake is washed with permeate (10 kg) and the effluent is combined with the filtrate.

Following the additional microfiltration, the filtrate is concentrated (by the baromembrane method) on a membrane with a pore size of 0.04 µm. This yields 5 kg of the concentrated product containing 1.8% (by weight) of sodium deoxyribonucleate. Sodium deoxyribonucleate is precipitated from the concentrated product by ethanol. The precipitate is separated by centrifugation and dried at 60°C for 4 hours.

This yields 66 g of sodium deoxyribonucleate having the following characteristics: molecular weight of 500 kDa, protein content of 0.1%, RNA + polysaccharides content of 0.2%, moisture of 5.5%, hyperchromic effect of 46%. The calculated amount of the powder (needed to prepare a 1.5% solution) is weighed out under aseptic conditions and dissolved in a 0.95% sterile solution of sodium chloride. The resulting solution is then aseptically poured into vials in therapeutic doses.

The analytical passport of the obtained preparation:
Identity - conforms;
Chromacity - colorless;
Transparency - conforms;
Sterility - sterile;
Toxicity - nontoxic;
Pyrogenicity - apyrogenic;
Nativity index - 2.3;
Content of sodium deoxyribonucleate - 0.015 g/mL;
pH - 7.9;
Content of sodium chloride - 0.95%;
Hyperchromic effect - 46%;
RNA content - 0.2%;
Protein content - 0.1%.

**Example 3.** 3 kg of defrosted and purified salmon milt roe are disintegrated and homogenized in 6 L of a citric-salt solution at 1,200 rpm. The homogenate is then centrifuged in a refrigerated sedimentation centrifuge at 3,500 rpm, and the supernatant is decanted and removed. The decantation and removal of the supernatant are repeated four times. The homogenate is then charged into a vessel with a citric-salt solution (21 L) and 3% solution of sodium dodecyl sulfate in 45% ethanol (5.7 kg) is added. The obtained mass is incubated at T=55-60°C for 2 hours under continuous agitation. Following this, a 5M sodium chloride solution (43 kg) is added and the reaction mass is again incubated under agitation at T=60°C for further 2 hours. Following this, the reacting mass is cooled to T=10-15°C and exposed to ultrasound in baths with piezoceramic radiators operating at a vibration frequency of 21.7 to 23 kHz, vibration amplitude of 12 µm and generator capacity of 1.5-2 kW. The sonicated mass is then mixed with kieselgur at a ratio of 20:2. The liquid phase is separated using a suction filter and concentrated by the baromembrane method. Sodium deoxyribonucleate is precipitated from the concentrated product by 96% ethanol. This yields 225 g of sodium deoxyribonucleate having the following characteristics: molecular weight of 490 kDa, RNA + polysaccharides content of 0.2%, protein content of less than 0.1%, hyperchromic effect of 46%, moisture of up to 10.5%.

Thus, the claimed method for producing sterile pharmaceutical substance of sodium deoxyribonucleate makes it possible to produce the higher purity product (with reducing the ballast protein content, RNA + polysaccharides content and moisture content to not more than 0.1 %, 0.2% and 5.5%, respectively), having improved physical-chemical properties (nativity index, pyrogenicity, identity), reduce the use of expensive and environmentally hazardous reagents, increase the technological effectiveness of equipment and ease its maintenance (baths with piezeceramic radiators are safe in terms of toxic substance emission, more durable and can be better disinfected), obtain the product meeting stricter current requirements for pharmaceutical substances and preparations (the using of sterilizing equipment and compartments with aseptic conditions excludes any microbial contamination of the finished product and extends both the shelf-life of the substance and active life of the preparation). The moisture content of 5-5.5% is necessary for producing medicinal preparation, the substance with decreased water content is insoluble in water and aqueous solutions of sodium chloride.

The offered apparatus makes the possibility to carry out the claimed method for producing an apyrogenic immunotropic sterile preparation for intravenous and painless intramuscular administration.

Table 1 shows characteristics of the prototype and the preparation obtained according with the present invention. As it is followed from the data, shown in the table, the product obtained according with the present invention is superior to its prototype in terms of physical-chemical characteristics.

Because of high purity and increased nativity index, the obtained immunomodulator is characterized by high immunotropic activity and restores the immune status at both cellular and humoral levels and can be used for intravenous administration in patients. Intramuscular administration of the preparation is painless. In additional to high therapeutic activity the preparation is very safe and can be used as a preventive medicinal preparation for restoration of the immune status of human. All existent substances of sodium deoxyribonucleate can't be used for producing the preparation for intravenous administration.

**Table 1**

| Key characteristics | Values (prototype) RU No.2236853 | Values of claimed invention |
|---|---|---|
| Molecular weight | 270-500 kDa | 270-500 kDa |
| Content of the basic | over 90% | 99.6-100% |
| substance matter | | |
| Nativity index | 2 | 2.3-2.4 |
| Protein content | up to 0.5% | 0.1 % |
| RNA content | up to 2% | 0.2% |
| pH | 6.2 | 7.9-8 |
| Hyperchromic effect | 42% and over | over 42% |
| Pyrogenicity | Apyrogenic | Apyrogenic |
| Microbiological purity | 2.2B | 1.2B |
| Sterility | Sterile | Sterile |
| Content of sodium | 0.08-0.12% | 0.9-0.95% |
| chloride | | |

Table 2 shows the comparative therapeutic effect of Reaferon, Interleukin-2 and the immunomodulator according with claimed invention.

**Table 2**

| **Illness** | **Quantity of patients (%) which are sensitive to** | | | |
|---|---|---|---|---|
| | **Immunomodulator according with invention** | **Derinat** | **Reaferon** | **Interleykin- 2** |
| Oncology | 90 | 70 | 20 | 60 |
| Hepatitis C | 80 | 70 | 40 | 50 |
| Genital herpes | 90 | 70 | 40 | 60 |
| Urology illness | 90 | 67 | 30 | 67 |
| Immunosuppression | 98 | 90 | 70 | 30 |
| Scatter sclerosis | 85 | 75 | 50 | 50 |
| Apparently healthy | 100 | 100 | 30 | 60 |

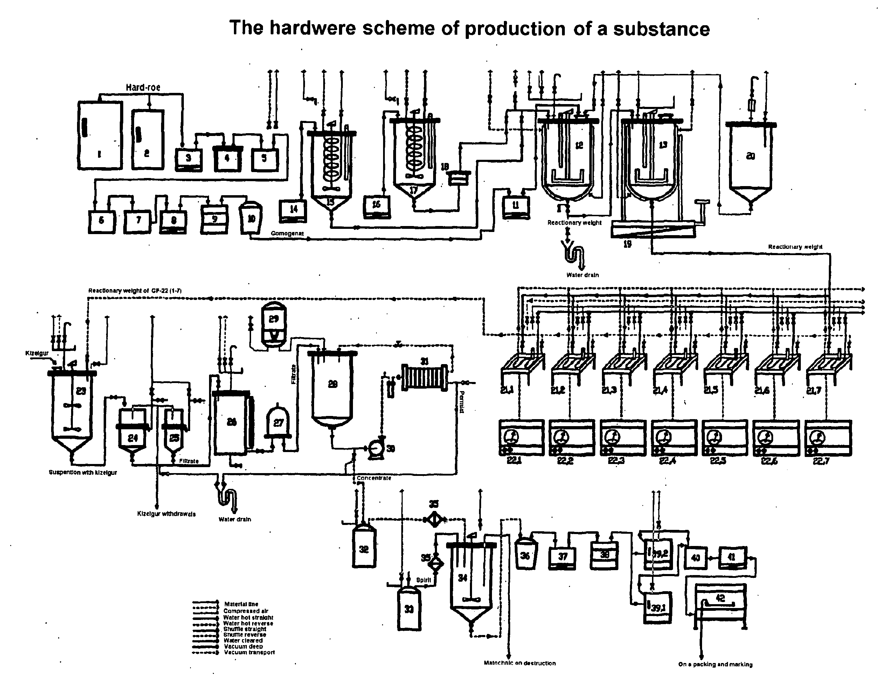

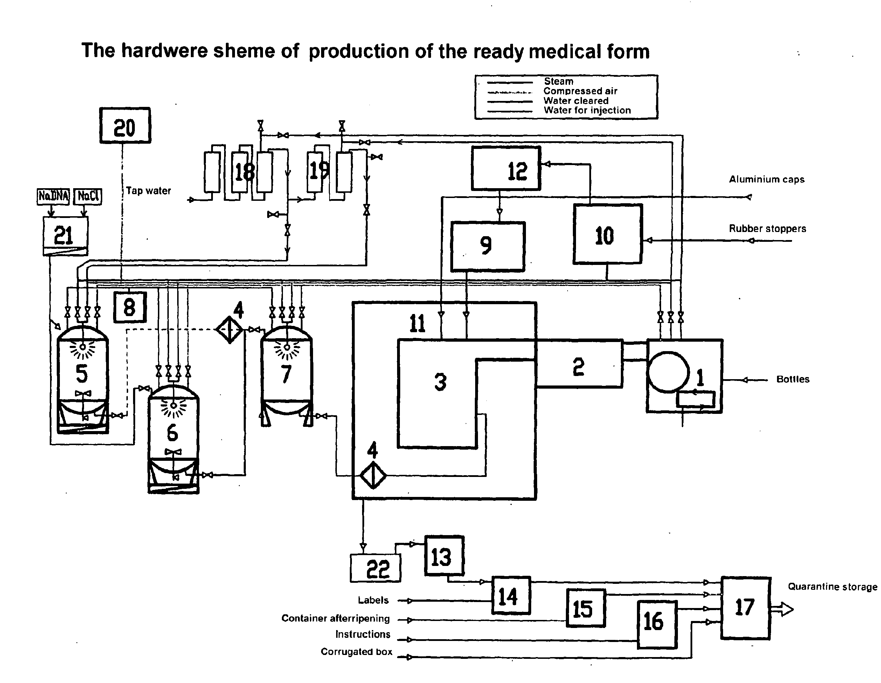

## Claims

1. A method for producing an immunotropic apyrogenic preparation, which is sodium salt of the native low-molecular deoxyribonucleic acid, comprising homogenizing salmon or sturgeon milt roe in an aqueous citric-salt solution, centrifuging, removing the supernatant, repeatedly homogenizing, incubating, adding sodium chloride solution, cooling reaction mass, treating with ultrasound, separating the liquid phase by sorbent, eluting sorbent by permeate, microfiltrating the eluate combined with liquid phase, concentrating, precipitating sodium deoxyribonucleate by ethanol, centrifuging to separate the liquid phase, drying, dissolving and pouring out, **characterized in that** the raw material is purified, degreased and disintegrated, and first homogenization is carried out at 12,000 rpm, and centrifugation in refrigerated sedimentation centrifuge is carried out at 3,500, the repeated homogenization is carried out 3-4 times with adding a citric-salt solution, which is followed by centrifugation to produce an air-fat foam, which is decanted, and incubation with a 3-6% of detergent under continuous agitation at 55-60°C with subsequent treating by sodium chloride solution and incubating the reaction mass at the same temperature for 2 hours; the reaction mass is cooled preferably to 10-15°C and treated with ultrasound in baths with piezoceramic radiators operating at a vibration frequency of 21.7 to 23 kHz, vibration amplitude of 9 µm and generator capacity of 1.5-2 kW; the drying of obtained substance is carried out under aseptic conditions at 40-60°C for 4-6 hours, the obtained white powder with a content of the basic substance matter of 99.6-100%, nativity index of over 2.2 and residual moisture of 5.5% is dissolved in 0.9-0.95% aqueous solution of sodium chloride to given concentrations and the resulting solution is aseptically poured out in therapeutic doses at pH 7.9-8.0, the produced preparation being unified immunomodulator, and this preparation can be used for intravenous or painless intramuscular administration.

2. The method of claim 1, **characterized in that** the centrifugation of homogenate at 3,500 rpm is carried out in a refrigerated centrifuge.

## Patentansprüche

1. Ein Verfahren zur Herstellung einer immunotropischen apyrogenen Vorbereitung, die Natriumsalz der nativen niedermolekularen Desoxyribonukleinsäure ist, und die die Homogenisierung der Fischmilch aus Salm oder Stör in einer wässrigen zitronen-salzigen Lösung, die Zentrifugierung, die Entfernung des Überstands, die Wiederholung der Homogenisierung, die Inkubation, die Zugabe von Natriumchloridlösung, das Abkühlen der Reaktionsmasse, die Behandlung mit Ultraschall, das Trennen der flüssigen Phase durch Sorbent, das Eluieren des Sorbents durch Permeat, das Mikrofiltern des Eluats, gemeinsam mit der flüssigen Phase, die Konzentrierung, das Niederschlagen des Natriumdeoxyribonucleate durch Äthanol, die Zentrifugierung, um die flüssige Phase abzuscheiden, das Trocknen, das Auflösen und das Ausschließen beinhaltet,
**dadurch gekennzeichnet, dass**
der Rohstoff gereinigt, abgefettet und aufgelöst wird, und die erste Homogenisation bei 12.000 U/min durchgeführt wird, und die Zentrifugierung in gekühlter Sedimentzentrifuge bei 3.500 durchgeführt wird, die wiederholte Homogenisation 3-4 mal mit der Zugabe von einer zitronen-salzigen Lösung durchgeführt wird, die von der Zentrifugierung gefolgt wird, um einen luftfettigen Schaum zu erzeugen, der abgegossen wird, und weiter die Inkubation mit 3-6% Reinigungsmittel unter ununterbrochenes Rühren bei 55-60°C mit anschließender Behandlung durch Natriumchloridlösung und die Inkubation der Reaktionsmasse bei der gleichen Temperatur 2 Stunden lang durchgeführt wird; die Reaktionsmasse wird vorzugsweise zu 10-15°C abgekühlt und mit Ultraschall im Bad mit piezokeramischen Heizkörpern behandelt, die bei einer Erschütterungsfrequenz von 21.7 bis 23 kHz, einer Erschütterungsamplitude von 9 µm und bei Generatorleistung von 1.5-2 Kilowatt funktionieren; die Trocknung der gewonnenen Substanz wird unter aseptischen Bedingungen bei 40-60°C 4-6 Stunden lang durchgeführt, das gewonnene weiße Pulver, mit einem Inhalt der grundlegenden Substanz von 99.6-100%, mit nativem Index von über 2.2 und Restfeuchtigkeit von 5.5%, wird in 0.9-0.95% wässeriger Lösung des Natriumchlorids zu den gegebenen Konzentrationen aufgelöst und die resultierende Lösung wird aseptisch in therapeutische Dosen bei pH 7.9-8.0 eingegossen, wobei die hergestellte Vorbereitung, die vereinheitliches Immunomodulator ist, für intravenöse oder schmerzlose intramuskulöse Einspritzung benutzt werden kann.

2. Die Methode von Anspruch 1, **dadurch gekennzeichnet, dass** die Zentrifugierung des Homogenats bei 3.500 U/min in einer gekühlten Zentrifuge durchgeführt wird.

## Revendications

1. Procédé de production d'un immun modulateur apyrogénique, qui représente un sel de l'acide désoxyribonucléique, bas moléculaire, comprenant une homogénéisation de caviar de saumon et d'esturgeon d'origine naturelle dans une solution aqueuse de sel limonène, centrifugation, suppression du superflu, homogénéisation répétée, incubation, addition de chlorure de sodium en solution, refroidissement de la masse réactionnelle, traitement avec ultrason, séparation de la phase liquide du sorbent, précipitation du sorbent, microfiltration du dépôt combiné avec la phase liquide, concentration, précipitation du sodium désoxyribonucléique avec de l'éthanol, centrifugation pour séparer la phase liquide, séchage et enlèvement, **caractérisé en ce que** les matires primaires sont purifiées, dégraissées et morcelées et une homogénéisation est menée à 12,000 rpm, et une centrifugation dans un centrifuge sédimentaire réfrigérée est menée à 3,500, la homogénéisation répétée est menée 3-4 fois en ajoutant une solution de sel de l'acide limonène, qui est suivi d'une centrifugation pour produire une écume épaisse, qui est ensuite décantée, et incubation avec 3-6% détergent à agitation incessamment à une température de 55-60°C et un traitement après avec une solution de chlorure de sodium et incubation de la réaction à la même température pendant deux heures; la masse réactionnelle est réfrigérée préférablement jusqu'à 10-15°C et est traitée avec ultrason dans des bains avec des radiateurs piézocéramiques opérant à des vibrations de fréquences de 21.7 to 23 kHz, amplitude des vibrations de 9 µm et capacité du générateur de 1.5-2 kW; le séchage de la substance obtenue est mené dans des conditions aseptiques à une température de 40-60°C pendant 4-6 heures, le poudre blanc obtenu, contenant la substance de base à 99.6-100%, indice naturel plus de 2.2 et humidité résiduelle de 5.5% est dissous dans 0.9-0.95% solution aqueuse de chlorure de sodium à des concentrations données et la solution obtenue est aseptiquement versée en doses thérapeutiques à pH 7.9-8.0, le produit préparé étant nommé immun modulateur, et ce produit peut être utilisé pour des administrations orales intraveineuses ou des administrations intramusculaires sans douleur.

2. Procédé selon la revendication 1, **caractérisé en ce que** la centrifugation du produit homogène à 3,500 rpm est menée dans une centrifuge réfrigérée.
